# EUROPEAN PATENT SPECIFICATION

(11) **EP 2 823 318 B1**
(45) Date of publication and mention of the grant of the patent: **10.08.2016**
(21) Application number: 13708192.3
(22) Date of filing: 08.03.2013
(51) Int. Cl.: G01N 33/74

(54) **A METHOD FOR PREDICTING THE RISK OF GETTING A CARDIOVASCULAR EVENT IN A FEMALE SUBJECT**
VERFAHREN ZUR VORHERSAGE DES RISIKOS EINES HERZ-KREISLAUF-EREIGNISSES BEI EINER WEIBLICHEN PATIENTIN
MÉTHODE DE PRÉDICTION DU RISQUE DE SURVENUE D'UN ÉVÉNEMENT CARDIOVASCULAIRE CHEZ UN SUJET DE SEXE FÉMININ

(30) Priority: 08.03.2012 EP 12158678; 08.03.2012 US 201261608376 P; 20.04.2012 EP 12165057
(43) Date of publication of application: 14.01.2015
(73) Proprietor: SphingoTec GmbH, 16761 Henningsdorf (DE)
(72) Inventor: BERGMANN, Andreas, 13645 Berlin (DE); MELANDER, Olle, S-205 02 Malmö (SE)
(74) Representative: Kilger, Ute
(86) International application number: PCT/EP2013/054799
(87) International publication number: WO 2013/132088

(56) References cited:
- WO-A1-2010/128071
- US-A1- 2005 130 230
- ERNST A ET AL: "Proneurotensin 1-117, a stable neurotensin precursor fragment identified in human circulation", PEPTIDES, ELSEVIER, AMSTERDAM, vol. 27, no. 7, 1 July 2006 (2006-07-01), pages 1787-1793, XP027957440, ISSN: 0196-9781 [retrieved on 2006-07-01]
- DATABASE EMBASE [Online] ELSEVIER SCIENCE PUBLISHERS, AMSTERDAM, NL; October 2004 (2004-10), LIU R -M ET AL: "Correlation of plasma neuropeptide Y and neurotensin with cardiac functional damage and prognosis in essential hypertension patients", XP002677074, Database accession no. EMB-2005023506 & CHINESE JOURNAL OF CLINICAL REHABILITATION 200410 CN, vol. 8, no. 30, October 2004 (2004-10), pages 6794-6795, ISSN: 1671-5926
- MELANDER OLLE ET AL: "Plasma Proneurotensin and Incidence of Diabetes, Cardiovascular Disease, Breast Cancer, and Mortality", JAMA THE JOURNAL OF THE AMERICAN MEDICAL ASSOCIATION, AMERICAN MEDICAL ASSOCIATION, US, vol. 308, no. 14, 1 October 2012 (2012-10-01), pages 1469-1475, XP009165659, ISSN: 0098-7484

## Description

Subject matter of the present invention is:
(i) A method for predicting the risk of getting a cardiovascular event in a female subject comprising:
   - determining the level of pro-neurotensin 1-117 in a bodily fluid obtained from said female subject; and
   - correlating said level of pro-neurotensin 1-117 with the risk of getting a cardiovascular event, wherein an elevated level is predictive of an enhanced risk of getting a cardiovascular event,
   wherein said cardiovascular event is an acute cardiovascular event selected from the group comprising myocardial infarction, stroke, acute heart failure and cardiovascular death related to myocardial infarction, stroke or acute heart failure,
   and wherein the level of pro-neurotensin 1-117 in a bodily fluid is the fasting level.
(ii) A method according to item (i), wherein said female subject has no history of diagnosis of an acute cardiovascular event at the time the sample of bodily fluid is taken from said female subject.
(iii) A method according to items (i) and (ii), wherein at the time the sample of bodily fluid is taken from said female subject, said female subject has been diagnosed as having a cardiovascular disease or diabetes.
(iv) A method according to item (iii), wherein the female subject has been diagnosed as having a cardiovascular disease selected from the group comprising heart failure, atherosclerosis, and hypertension.
(v) A method according to item (iii), wherein the female subject has been diagnosed as having at diabetes type II.
(vi) A method according to items (i) to (v) wherein in addition the level of one or both of the following markers is determined: pro-BNP or fragments or precursors thereof having at least 5 amino acids and/or C-reactive protein (CRP).
(vii) A method according to items (i) to (vi), wherein additionally at least one clinical parameter is determined selected from the group comprising: age, systolic blood pressure, diastolic blood pressure, antihypertensive treatment, body mass index, presence of diabetes mellitus, current smoking.
(viii) A method according to any of items (i) to (vii), wherein said method is performed more than once in order to monitor the risk of getting a cardiovascular event in a female subject.
(ix) A method according to item (viii), wherein said monitoring is performed in order to evaluate the response of said female subject to preventive and/or therapeutic measures taken.
(x) A method according to any of items (i) to (ix) in order to stratify said female subjects into risk groups.
(xi) Use of a device, e.g. point-of-care device for performing a method according to any of items (i) to (x).

The term "elevated level" means a level above a certain threshold level.

Neurotensin is a 13-amino acid neuropeptide derived from the prepro-neurotensin precursor and stochiometrically released together with the stable 117-amino acid peptide pro-neurotensin (P-NT) and the mature hormone binds to three different receptors, neurotensin receptor 1 and 2 (Ntsrl and Ntsr2), which are G-protein coupled receptors and neurotensin receptor 3 (Ntsr3) which is non-G-protein coupled and also known as Sortillin-1 (SORT1).

Neurotensin is released peripherally from the small intestine as well as centrally from the hypothalamus. The peripheral secretion of neurotensin is stimulated by food-intake, especially by fat, and is known to regulate gastrointestinal motility and pancreatic and biliary secretion. Interestingly, neurotensin is implicated in appetite control as an anorectic hormone as it acutely reduces food intake following both central (intracerebroventricular) and peripheral (intraperitoneal) injection in rats, an effect which seems mainly mediated through the neurotensin-1 receptor (Ntsr1). In obese as compared to normal-weight human subjects, postprandial plasma neurotensin concentration was reduced following a liquid fatty meal (Widen et al 1992, Reg peptides; Plasma concentrations of regulators peptides in obesity following modified sham feeding (MSF) and a liquid test meal), suggesting regulation of neurotensin secretion is disturbed in obesity. However, no large study has investigated if and how neurotensin is related to measures of obesity. Interestingly, P-NT significantly increases after gastric by-pass (Roux-en-Y), an operation shown to lead to normoglycemia in the majority of obese type 2 diabetes patients, but it is not known whether neurotensin is implicated in the development diabetes mellitus in general. Furthermore, the neurotensin system has been implicated in development of coronary artery disease and myocardial infarction as variation of the Ntsr3 (SORT1) gene is one of the strongest common coronary artery diseases susceptibility genes known in humans.

The mechanistic link between obesity and cancer is largely unknown, however, one of the dominating theories is that excess of fat deposits leads to increased peripheral aromatization of androgens and thus elevated circulating estrogen levels. In addition, one of the hallmarks of obesity, hyperinsulinemia, has been shown to inhibit hepatic production of Sexual Hormone Binding Globulin (SHBG), thus increasing bioavailable levels of both estrogens and androgens suggesting ways through which obesity may increase the risk of common forms of sex-hormone driven forms of cancer such as breast and prostate cancer. Interestingly, both neurotensin and Ntsr1 expression is common in malignant ductal breast cancer tumors and experimentally pharmacological blockade or RNA silencing of the NTSR1 reduces tumour growth in mice.

The level of expression of neurotensin receptor 1 (NTSR1) in breast cancer cells has been used for determining the prognosis of a subject suffering from breast cancer (US 2011/0305633). Further, it is stated in by the same authors that no clear correlation has been described today between circulating neurotensin and the stages of pancreas, prostate, or medullar thyroid tumors probably due to rapid clearance by the liver. Interestingly, it was found that in a series of 51 patients with invasive ductal breast cancer 91 % of all tumors were positive for neurotensin receptor 1 (NTSR1) but only 31 % of all tumors were positive for neurotensin in said tissue (Souaze et. Al. Cancer Research 2006; 66: (12) pages 6243-6249.

There is some evidence that neurotensin and neurotensin receptors participate in cancer growth, in particular in lung cancer, pancreatic cancer and colon cancer (Carraway et al.; Peptides 27 (2006) 2445-2460). It has been reported that levels of NT in sera of patients with pancreatic cancer were significantly enhanced (Picheon et al, Anticancer Research 1999; 19; 1445-50).Interestingly this group found that NT levels fell with progression of the disease for both prostate an pancreatic cancer. In contrast, thereto, Meggiato et al; Tumori 1996; 82; 592-5; found that plasma levels of NT were normal in pancreatic cancer but elevated in case where pancreatitis was diagnosed.

The use of vasoactive peptides for prediction of cancer risks in males has been reported by belting et al, Cancer, Epidemiology, Biomarkes & Prevention. MR-pro-ANP , MR-pro-ADM and copeptin was measured in the fasting plasma from participants of the Malmö Diet and Cancer Study that were free from cancer prior to the baseline exam in 1991 to 1994 (1768 males and 2293 females) The Authors stated that among females, there was no relationship between biomarkers and cancer incidence.

CRP and Pro-BNP are known predictors of cardiovascular events in the population (Melander et. Al, JAMA. 2009;302(1):49-57). There is now information about any gender difference of the predictive power CRP and Pro-BNP for CVD endpoints.

EP2427764 discloses methods of determining the concentration of vasoactive hormones and their precursors and fragments thereof in a sample derived from a bodily fluid of a patient and the stratification of patients suffering from a disease related to endothelial function/dysfunction.

A subject of the present invention was to investigate the prognostic and diagnostic power of NT for predicting the risk of getting a cardiovascular event in a female subject to address this issue, we measured stable fragments of pro-neurotensin 1-117 in fasting plasma in said Swedish prospective cohort study (Malmö Diet and Cancer Study, see Melander et. al, JAMA. 2009;302(1):49-57) and related baseline level of this biomarker to cardiovascular events during 15 years of follow-up.

Surprisingly, it has been shown that pro-neurotensin 1-117 is a powerful and highly significant biomarker for woman for predicting the risk of getting a cardiovascular event

Thus, subject of the present invention is a method for predicting the risk of getting a cardiovascular event in a female subject comprising:
- determining the level of pro-neurotensin 1-117 in a bodily fluid obtained from said female subject; and
- correlating said level of pro-neurotensin 1-117 with the risk of getting a cardiovascular event, wherein an elevated level is predictive of an enhanced risk of getting a cardiovascular event, wherein said cardiovascular event is an acute cardiovascular event selected from the group comprising myocardial infarction, stroke, acute heart failure and cardiovascular death related to myocardial infarction, stroke or acute heart failure, and wherein the level of pro-neurotensin 1-117 in a bodily fluid is the fasting level.

The level of pro-neurotensin 1-117 in a bodily fluid obtained from said female subject that is predictive for the risk of getting a cardiovascular event in said female subject may is released from the small intestine. The release of neurotensin from the small intestine is stimulated by food intake, especially by fat, and is known to regulate gastrointestinal motility and pancreatic and biliary secretion. Pro-neurotensin 1 -117 is used as a surrogate marker for the released neurotensin as neurotensin and pro-neurotensin 1 -117 are released in equimolar amounts from pro-neurotensin.

It is the surprising finding of the present invention that the peripheral secretion of pro-neurotensin 1-117 is indicative for the susceptibility of a female subject to get a cardiovascular event. Thus, dietary measures as reduction of fat uptake may lower said risk in said female subject.

Data obtained in present study revealed also a correlation between the risk of getting a cardiovascular event in male subjects with the level of pro-neurotensin 1-117 in a bodily fluid obtained from said male subject; this correlation however, was not that significant for the present data. Thus, we assume that there is a value for the method according to the invention also for male subjects but the observed effect was not that strong for males in the present study.

The term "subject" as used herein refers to a living human or non-human organism. Preferably herein the subject is a human subject.

The correlation between the level of pro-neurotensin 1-117 in a bodily fluid obtained from said female subject and the risk of getting a cardiovascular event is continuous, i.e. the higher the level the higher the risk. This can be seen from the data e.g. in Table 17. In comparison to the first quartile the second, third and forth quartile exhibits higher Hazard Risks respectively.

For the sake of practicability the person skilled in the art may use threshold(s).

Thus, the term "elevated level" may mean a level above a threshold level.

In one embodiment of the invention the level of pro-neurotensin in a bodily fluid obtained from a female subject is the fasting level of pro-neurotensin 1-117. Fasting level means no food uptake 12 h prior blood sampling.

A bodily fluid may be selected from the group comprising blood, serum, plasma, urine, cerebro spinal liquid (csf), and saliva.

In one embodiment of a method according to the present invention said female subject has no history of diagnosis of an acute cardiovascular event at the time the sample of bodily fluid is taken from said female subject.

In another embodiment of a method according to the present invention said female subject has been diagnosed as having at a cardiovascular disease or diabetes at the time the sample of bodily fluid is taken from said female subject.

In a specific embodiment said cardiovascular disease at the time the sample of bodily fluid is taken from said female subject may be selected from the group comprising heart failure, atherosclerosis, and hypertension.

The present data suggest a strong correlation between the level of pro-neurotensin or fragments thereof with a cardiovascular event in woman with no prevalent diabetes, no prevalent breast cancer and no prevalent cardiovascular disease.

The present data also suggest a strong correlation between the level of pro-neurotensin or fragments thereof with a cardiovascular event in hypertensive woman, which is a common high-risk group for cardiovascular disease.

Furthermore, the present data also suggest a strong correlation between the level of pro-neurotensin or fragments thereof with a cardiovascular event in normotensive woman. Further, the present data suggest a strong correlation between the level of pro-neurotensin or fragments thereof with a cardiovascular event in diabetic woman.

In another specific embodiment of the invention at the time the sample of bodily fluid is taken from said female subject, said female subject has been diagnosed as having at diabetes Typ II. In a specific embodiment of the invention the prediction of a first adverse event in a subject or the identification of a subject having an enhanced risk for getting a first adverse event is improved by additionally determining and using the level of at least one further marker selected from the group comprising: CRP, LpLA2, Cystatin C and natriuretic peptides of the A- and the B-type as well as their precursors and fragments thereof including ANP, proANP, NT-proANP, MR-proANP, BNP, proBNP, NT-proBNP triglycerides, HDL cholesterol or subtractions thereof, LDL cholesterol or subtractions thereof, GDF15, ST2.

In a very specific embodiment of the method according to the invention in addition to the level of pro-neurotensin 1-117 the level of one or both of the following marker is determined and used: proBNP or fragments or precursors thereof having at least 5 amino acids and/or C-reactive protein (CRP).

In another specific embodiment of the invention additionally at least one clinical parameter is determined selected from the group comprising: age, systolic blood pressure, diastolic blood pressure, antihypertensive treatment, body mass index, presence of diabetes mellitus, current smoking.

Cardiovascular events (CVD) were defined as coronary events or fatal or nonfatal stroke. Events were identified through linkage of the 10-digit personal identification number of each Swedish citizen with 3 registries: the Swedish Hospital Discharge Register, the Swedish Cause of Death Register, and the Stroke in Malmö register. Myocardial infarction was defined on the basis of International Classification of Diseases, 9th and 10th revisions (ICD-9 and ICD-10) codes 410 and 121, respectively. Fatal or nonfatal stroke was defined using codes 430, 431, 434, and 436 (ICD-9) and 160, 161, 163, and 164 (ICD-10).

The definition of diabetes is as follows: a history of physician diagnosis or being on anti-diabetic medication or having a fasting whole blood glucose >/= 6.1 mmol/1 (note this is = 7.0 mmol/l in plasma) at the baseline examination.

The definition of normotensivel high blood pressure (HBP) is as follows:
HBP: Systolic BP>/=140 mmHg Diastolic BP >/-90 mmHg or being on antihypertensive medications. Subjects having normal blood pressure (BP) are all other subjects, i.e subjects with Systolic BP<140 mmHg or Diastolic BP <90 mmHg or not being on antihypertensive medications.

Fragments of pro-neurotensin that may be determined in a bodily fluid may be e.g. selected from the group of the following fragments, wherein pro-neurotensin 1-117 is used according to the claims:
SEQ ID NO: 1 (pro-neurotensin 1-147) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYILKRQLY ENKPRRPYIL KRDSYYY
SEQ ID NO: 2 (pro-neurotensin 1-125 (large neuromedin N)) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVI KR KIPYIL
SEQ ID NO: 3 (neuromedin N:)
   KIPYIL
SEQ ID NO: 4 (neurotensin)
   pyroQLYENKPRRP YIL
SEQ ID NO: 5 (pro-neurotensin 1-117) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVI
SEQ ID NO: 6 (pro-neurotensin 1-132) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYILKRQLY EN
Seq ID No 7: (pro-neurotensin 1-125) 
SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYIL
SEQ ID NO: 8 (pro-neurotensin 120-140)
   KIPYILKRQL YENKPRRPYI L
SEQ ID NO: 9 (pro-neurotensin 120-147)
   KIPYILKRQL YENKPRRPYIL KRDSYYY
SEQ ID NO: 10 (pro-neurotensin 128-147)
   QLYENKPRRP YILKRDSYYY

In the methods according to the present invention the level of pro-neurotensin 1-117 is determined.

In a specific embodiment the level of pro-neurotensin 1-117 is measured with an immunoassay. More specifically an immunoassay is used as described in Ernst et al. Peptides 27 (2006) 1787-1793. An immunoassay that may be useful for determining the level of pro-neurotensin 1-117 may comprise the steps as outlined in Example 2. All thresholds and values have to be seen in correlation to the test and the calibration used according to Example 2. A person skilled in the art may know that the absolute value of a threshold might be influenced by the calibration used. This means that all values and thresholds given herein are to be understood in context of the calibration used in herein (Example 2). A human P-NT-calibrator is available by ICI-Diagnostics, Berlin, Germany. Alternatively, the assay may also be calibrated by synthetic or recombinant P-NT 1-117 or fragments thereof (see also Ernst et. al, 2006).

The threshold for determining the risk of getting a cardiovascular event in a female subject according to the methods of the present invention is above 78 pmol/1 PNT, preferred 100 pmol/1, more preferred 150 pmol/1. In a specific embodiment said threshold is about 100 pmol/1. These thresholds are related to the above mentioned calibration method. A P-NT value above said threshold means that the subject has an enhanced risk of getting a cardiovascular event.

Generally, in the prediction of the risk of a subject for contracting cardiovascular events an improvement can be achieved by additionally determining and using the level of at least one laboratory parameter or further marker selected from the group comprising fasting blood or plasma glucose, triglycerides, HDL cholesterol or subtractions thereof, LDL cholesterol or subfractions thereof, Cystatin C, Insulin, CRP, vasopressin or its precursors or fragments thereof and BNP or its precursors or fragments thereof.

In a specific embodiment of the method according to the invention additionally at least one clinical parameter is determined selected from the group comprising age, systolic blood pressure, diastolic blood pressure, antihypertensive treatment (AHT), body mass index, presence of diabetes mellitus, and current smoking.

Subject matter of the present invention is further a method for predicting the risk of getting a cardiovascular event in a subject or identifying a subject having an enhanced risk for getting a cardiovascular event, wherein the level of pro-neurotensin 1-117 either alone or in conjunction with other prognostically useful laboratory or clinical parameters is used for the prediction of a subject's risk for getting a cardiovascular event by a method which may be selected from the following alternatives:
- Comparison with the median of the level of pro-neurotensin 1-117 in an ensemble of pre-determined samples in a population of "healthy" or "apparently healthy" subjects,
- Comparison with a quantile of the level of pro-neurotensin 1-117 in an ensemble of pre-determined samples in a population of "healthy" or "apparently healthy" subjects,
- Calculation based on Cox Proportional Hazards analysis or by using Risk index calculations such as the NRI (Net Reclassification Index) or the IDI (Integrated Discrimination Index).

In one embodiment of the method according to the invention said method is performed more than once in order to monitor the risk of getting a cardiovascular event in a female subject.

In another embodiment of the method according to the invention said monitoring is performed in order to evaluate the response of said female subject to preventive and/or therapeutic measures taken.

In another embodiment of the method according to the in order invention the method is used to stratify said female subjects into risk groups.

Also encompassed by the present invention is the use of a point-of-care device for performing a method according to the invention.

Also disclosed, but not claimed, is an assay and/or kit for performing a method according to the invention.

Disclosed, but not claimed, is also a binder to neurotensin or to a neurotensin receptor, for the use in prevention or therapy of a cardiovascular event in a female subject. Said binder reduces the bioactivity of neurotensin to 70 % or less.

The disclosed but not claimed binder to neurotensin may be selected from the group consisting of antibodies e.g. IgG, a typical full-length immunoglobulin, or antibody fragments containing at least the F-variable domain of heavy and/or light chain as *e.g*. chemically coupled antibodies (fragment antigen binding) including but not limited to Fab-fragments including Fab minibodies, single chain Fab antibody, monovalent Fab antibody with epitope tags, e.g. Fab-V5Sx2; bivalent Fab (mini-antibody) dimerized with the CH3 domain; bivalent Fab or multivalent Fab, e.g. formed via multimerization with the aid of a heterologous domain, e.g. via dimerization of dHLX domains,*e*.*g*. Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, *e.g.* from a different class than G; single-domain antibodies, *e.g.* nanobodies derived from camelid or fish immunoglobulines.

The disclosed, but not claimed binder to a neurotensin receptor may be selected from the group consisting of antibodies e.g. IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g*. Fab-V5Sx2; bivalent Fab (mini-antibody) dimerized with the CH3 domain; bivalent Fab or multivalent Fab, *e.g.* formed via multimerization with the aid of a heterologous domain, *e.g*. via dimerization of dHLX domains,*e*.*g*. Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, *e.g*. from a different class than G; single-domain antibodies, e.g. nanobodies derived from camelid or fish immunoglobulines, or a peptide antagonist e.g. [D-Trp¹¹]-Neurotensin, [Tyr(Me)¹¹]-Neurotensin (e.g. described by Quiron et al.), or a non-peptide antagonist, e.g. Levocabastine, SR-48692 (NTS1 selective), SR-142948 (unselective), SR-142948A, CP 96345, [3H]SR-48692, SR 48692, SR-48527 and SR-49711, or a binder scaffold e.g. tetranectin-based non-Ig scaffolds (e.g. described in US 201010028995), fibronectin scaffolds (e.g. described in EP 1266 025; lipocalin-based scaffolds ((e.g. described in WO 2011/154420); ubiquitin scaffolds (e.g. described in WO 2011/073214), transferring scaffolds (e.g. described in US 2004/0023334), protein A scaffolds (e.g. described in EP 2231860), ankyrin repeat based scaffolds (e.g. described in WO 2010/060748), microproteins preferably microproteins forming a cystine knot) scaffolds (e.g. described in EP 2314308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1941867).

### Examples

### Example 1

### Development of Antibodies

### Peptides/ conjugates for Immunization:

Peptides for immunization were synthesized (JPT Technologies, Berlin, Germany) with an additional N-terminal Cystein residue for conjugation of the peptides to Bovine Serum Albumin (BSA). The peptides were covalently linked to BSA by using Sulfo-SMCC (Perbio-science, Bonn, Germany). The coupling procedure was performed according to the manual of Perbio.
Labelled antibody (LA) peptide (P-NT 1-19):
   H-CSDSEEEMKALEADFLTNMH-NH2
Solid phase antibody (SPA) peptide (P-NT 44-62):
   H-CNLNSPAEETGEVHEEELVA-NH2

The antibodies were generated according to the following method:
A BALB/c mouse were immunized with 100 µg Peptide-BSA-Conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100µl saline, given as one intraperitoneal and one intra venous injection.

Splenocytes from the immunized mouse and cells of the myeloma cell line SP2/0 were fused with 1 ml 50 % polyethylene glycol for 30 s at 37 °C. After washing, the cells were seeded in 96-well cell culture plates. Hybrid clones were selected by growing in HAT medium [RPMI 1640 culture medium supplemented with 20 % fetal calf serum and HAT-Supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined.

(Lane, R.D. "A short-duration polyethylene glycol fusiontechnique for increasing production of monoclonal antibody-secreting hybridomas", J. Immunol. Meth. 81: 223-228; (1985), Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996)).

### Monoclonal antibody production

Antibodies were produced via standard antibody production methods (Marx et al, Monoclonal Antibody Production, ATLA 25, 121, 1997,) and purified via Protein A-chromatography. The antibody purities were > 95% based on SDS gel electrophoresis analysis.

### Example 2

### Immunoassay for the quantification of human pro-neurotensin

The technology used was a sandwich coated tube luminescence immunoassay, based on Akridinium ester labelling.

Labelled compound (tracer): 100 µg (100 µl) LA (1 mg/ml in PBS, pH 7.4, was mixed with 10 µl Akridinium NHS-ester (1 mg/ml in acetonitrile, InVent GmbH, Germany) (EP 0353971) and incubated for 20min at room temperature. Labelled LA was purified by Gel-filtration HPLC on Bio-Sil SEC 400-5 (Bio-Rad Laboratories, Inc., USA) The purified LA was diluted in (300 mmol/1 potassiumphosphate, 100 mmol/1 NaCl, 10 mmol/1 Na-EDTA, 5 g/1 Bovine Serum Albumin, pH 7.0). The final concentration was approx. 800.000 relative light units (RLU) of labelled compound (approx. 20 ng labeled antibody) per 200 µl. Akridiniumester chemiluminescence was measured by using an AutoLumat LB 953 (Berthold Technologies GmbH & Co. KG).

Solid phase: Polystyrene tubes (Greiner Bio-One International AG, Austria) were coated (18h at room temperature) with SPA ((1.5 µg SPA/0.3 ml 100 mmol/1 NaCl, 50 mmol/1 tris/HCl, pH 7.8). After blocking with 5 % bovine serum albumine, the tubes were washed with PBS, pH 7.4 and vacuum dried.

### Calibration:

The assay was calibrated, using dilutions of P-NT-containing human serum. A pool of human sera with high P-NT-immunoreactivity (InVent Diagostika, Hennigsdorf, Germany) was diluted with horse serum (Biochrom AG, Deutschland) (assay standards).

The standards were calibrated by use of the human P-NT-calibrator (ICI-Diagnostics, Berlin, Germany). Alternatively, the assay may be calibrated by synthetic or recombinant P-NT 1-117 or fragments thereof (see also Ernst et. al, 2006).

### P-NT Immunoassay:

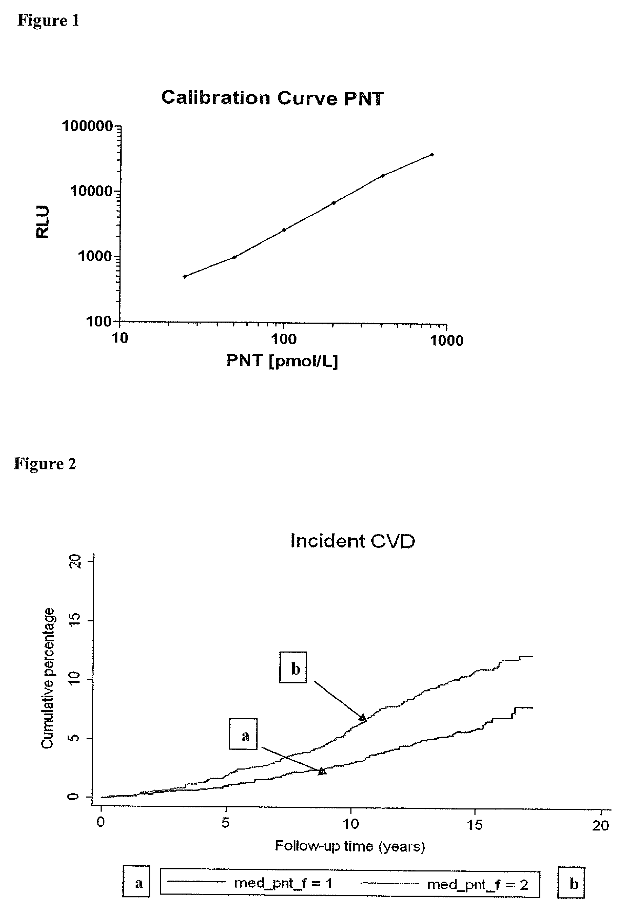

50 µl of sample (or calibrator) was pipetted into SPA coated tubes, after adding labelled LA (200µl), the tubes were incubated for 16-22 h at 18-25 °C. Unbound tracer was removed by washing 5 times (each 1 ml) with washing solution (20 mM PBS, pH 7.4, 0.1 % Triton X-100).
Tube-bound LA was measured by using the LB 953
Figure 1 shows a typical P-NT dose/ signal curve.

### Example 3: Population study

### Methods

We measured P-NT in fasting plasma from 4362 participants of the population based Malmö Diet and Cancer Study baseline exam in 1991-1994 (men age 58 ± 6 years and 59 % females). We used multivariable adjusted (all traditional cardiovascular risk factors, diabetes risk factors and in analyses of cancer also heredity for cancer) Cox proportional hazards models to relate baseline P-NT (hazard ratio per each standard deviation increase of log-transformed P-NT) to the time to the first event of each of the studied endpoints during a median follow-up time of more than 12 years. Endpoints were retrieved through the Swedish National Hospital Discharge Registry, the Swedish Myocardial Infarction Registry, the Stroke in Malmö Registry and the Swedish Cancer Registry. Retrieval of endpoints through these registries has been validated and found to be accurate.

### Clinical characteristics of the total study population

**Table 1**

| | | | |
|---|---|---|---|
| **Descriptive Statistics** | | | |

| | N | Mean | Std. Deviation |
|---|---|---|---|
| Age at MDCS screening | 4362 | 57.643 | 5.9797 |
| Systolic blood pressure (mmHg) | 4362 | 141.91 | 19.158 |
| Diastolic blood presure (mmHg) | 4362 | 87.02 | 9.501 |
| body-mass-index (weight/kgxkg) | 4362 | 25.7642 | 3.91173 |
| WAIST (cm) | 4361 | 83.56 | 12.791 |
| Glucose (mmol/1) | 4362 | 5.1826 | 1.33736 |
| Triglycerides (mmol/1) | 4362 | 1.3142 | .63660 |
| High density lipoprotein (mmol/1) | 4362 | 1.3908 | .37068 |
| Low density lipoprotein (mmol/1) | 4362 | 4.1632 | .98774 |
| P-INSULIN | 4280 | 7.889 | 7.6975 |
| PNT (pmol/1) | 4362 | 123.01743 | 76.746549 |
| Valid N (listwise) | 4279 | | |

**Table 2**

| **gender** | | | | | |
|---|---|---|---|---|---|
| | | Frequency | Percent | Valid Percent | Cumulative Percent |
| Valid | male | 1803 | 41.3 | 41.3 | 41.3 |
| | woman | 2559 | 58.7 | 58.7 | 100.0 |
| | Total | 4362 | 100.0 | 100.0 | |

**Table 3**

| **Q+Diary: Anti Hypertension Treatment (C02,C03,C07,C08,C09) at** baseline **according to questionnaire or diary book** | | | | | |
|---|---|---|---|---|---|
| | | Frequency | Percent | Valid Percent | Cumulative Percent |
| Valid | No | 3684 | 84.5 | 84.5 | 84.5 |
| | Yes | 678 | 15.5 | 15.5 | 100.0 |
| | Total | 4362 | 100.0 | 100.0 | |

**Table 4**

| **DIAB MELL** (**fb >6**.**0 or pos Q DM)** | | | | | |
|---|---|---|---|---|---|
| | | Frequency | Percent | Valid Percent | Cumulative Percent |
| Valid | no | 3993 | 91.5 | 91.5 | 91.5 |
| | yes | 369 | 8.5 | 8.5 | 100.0 |
| | Total | 4362 | 100.0 | 100.0 | |

**Table 5**

| **current smoker0** | | | | | |
|---|---|---|---|---|---|
| | | Frequency | Percent | Valid Percent | Cumulative Percent |
| Valid | .00 | 3212 | 73.6 | 73.6 | 73.6 |
| | 1.00 | 1150 | 26.4 | 26.4 | 100.0 |
| | Total | 4362 | 100.0 | 100.0 | |

**Table 6**

| **Clinical characteristics of females in the study** | | | |
|---|---|---|---|
| **Descriptive Statistics** | | | |
| | N | Mean | Std. Deviation |
| Age at MDCS screening | 2559 | 57.554 | 5.9403 |
| Systolic blood pressure (mm Hg) | 2559 | 140.50 | 19.311 |
| Diastolic blood presure (mm Hg) | 2559 | 85.65 | 9.117 |
| body-mass-index (weight/kgxkg) | 2559 | 25.5196 | 4.19083 |
| WAIST (cm) | 2559 | 76.99 | 10.245 |
| Glucose (mmol/1) | 2559 | 5.0418 | 1.21798 |
| Triglycerides (mmol/1) | 2559 | 1.2245 | .58404 |
| High density lipoprotein (mmol/1) | 2559 | 1.5123 | .36949 |
| Low density lipoprotein (mmol/1) | 2559 | 4.2016 | 1.04762 |
| P-INSULIN | 2512 | 7.223 | 5.4223 |
| PNT [pmol/L] | 2559 | 125.60633 | 77.681673 |
| Valid N (listwise) | 2512 | | |

**Table 7**

| **Q+Diary: Anti Hypertension Treatment (C02,C03,C07,C08,C09) at baseline according to questionnaire or diary book** | | | | | |
|---|---|---|---|---|---|
| | | Frequency | Percent | Valid Percent | Cumulative Percent |
| Valid | No | 2173 | 84.9 | 84.9 | 84.9 |
| | Yes | 386 | 15.1 | 15.1 | 100.0 |
| | Total | 2559 | 100.0 | 100.0 | |

**Table 8**

| **DIAB MELL (fb >6.0 or pos Q DM)** | | | | | |
|---|---|---|---|---|---|
| | | Frequency | Percent | Valid Percent | Cumulative Percent |
| Valid | no | 2396 | 93.6 | 93.6 | 93.6 |
| | yes | 163 | 6.4 | 6.4 | 100.0 |
| | Total | 2559 | 100.0 | 100.0 | |

**Table 9**

| **current smoker0** | | | | | |
|---|---|---|---|---|---|
| | | Frequency | Percent | Valid Percent | Cumulative Percent |
| Valid | .00 | 1906 | 74.5 | 74.5 | 74.5 |
| | 1.00 | 653 | 25.5 | 25.5 | 100.0 |
| | Total | 2559 | 100.0 | 100.0 | |

**Table 10**

| **Clinical characteristics of males in the study** | | | |
|---|---|---|---|
| **Descriptive Statistics** | | | |
| | N | Mean | Std. Deviation |
| Age at MDCS screening | 1803 | 57.769 | 6.0345 |
| Systolic blood pressure (mm Hg) | 1803 | 143.90 | 18.766 |
| Diastolic blood presure (mm Hg) | 1803 | 88.95 | 9.698 |
| body-mass-index (weight/kgxkg) | 1803 | 26.1113 | 3.44882 |
| WAIST (cm) | 1802 | 92.89 | 9.932 |
| Glucose (mmol/1) | 1803 | 5.3825 | 1.46780 |
| Triglycerides (mmol/1) | 1803 | 1.4416 | .68477 |
| High density lipoprotein (mmol/1) | 1803 | 1.2183 | .29669 |
| Low density lipoprotein (mmol/1) | 1803 | 4.1087 | .89336 |
| P-INSULIN | 1768 | 8.835 | 10.0090 |
| PNT [pmol/1] | 1803 | 119.34300 | 75.268054 |
| Valid N (listwise) | 1767 | | |

**Table 11**

| **Q+Diary: Anti Hypertension Treatment (C02,C03,C07,C08,C09) at baseline according to questionnaire or diary book** | | | | | |
|---|---|---|---|---|---|
| | | Frequency | Percent | Valid Percent | Cumulative Percent |
| Valid | No | 1511 | 83.8 | 83.8 | 83.8 |
| | Yes | 292 | 16.2 | 16.2 | 100.0 |
| | Total | 1803 | 100.0 | 100.0 | |

**Table 12**

| **DIAB MELL** (**fb >6.0 or pos Q DM)** | | | | | |
|---|---|---|---|---|---|
| | | Frequency | Percent | Valid Percent | Cumulative Percent |
| Valid | no | 1597 | 88.6 | 88.6 | 88.6 |
| | yes | 206 | 11.4 | 11.4 | 100.0 |
| | Total | 1803 | 100.0 | 100.0 | |

**Table 13**

| **current smoker0** | | | | | |
|---|---|---|---|---|---|
| | | Frequency | Percent | Valid Percent | Cumulative Percent |
| Valid | .00 | 1306 | 72.4 | 72.4 | 72.4 |
| | 1.00 | 497 | 27.6 | 27.6 | 100.0 |
| | Total | 1803 | 100.0 | 100.0 | |

### Results

### Cross sectional relationship between cardiometabolic risk factors and P-NT

The baseline characteristics of the study population are shown in Table 1. Women had slightly but significantly higher P-NT [median (interquartile range)] than men [109 (79-150) versus 99 (71-144) pmol/1] (P<0.001). The cross sectional relationship between P-NT and measures of obesity, cardiovascular risk factors and diabetes risk factors was generally weak with the strongest correlation being that with fasting insulin concentration in both gender (Supplementary Table 1). In a linear regression model with backward elimination and a retention P-value of 0.10, significant independent determinants of P-NT were smoking and fasting concentrations of insulin, glucose, and HDL (all positive) in women and smoking and fasting concentrations of insulin and HDL (positively related) and age and LDL (negatively related) in men (Table 2).

**Table 14**

| QUARTILES OF PNT IN ALL: | | | | |
|---|---|---|---|---|
| PNT [pmol/1] | | | | |
| Percentile Group of PNTpmolL | N | Median | Minimum | Maximum |
| 1 | 1091 | 60.22000 | 3.270 | 75.740 |
| 2 | 1090 | 89.29000 | 75.790 | 104.600 |
| 3 | 1092 | 122.67000 | 104.640 | 147.610 |
| 4 | 1089 | 190.03000 | 147.660 | 1154.520 |
| Total | 4362 | 104.62000 | 3.270 | 1154.520 |

**Table 15**

| QUARTILES OF PNT IN WOMEN: | | | | |
|---|---|---|---|---|
| PNT [pmol/1] | | | | |
| Percentile Group of PNTpmolL | N | Median | Minimum | Maximum |
| 1 | 639 | 62.37000 | 5.100 | 78.580 |
| 2 | 639 | 92.07000 | 78.610 | 108.770 |
| 3 | 641 | 125.07000 | 108.960 | 150.000 |
| 4 | 640 | 194.38500 | 150.050 | 1154.520 |
| Total | 2559 | 108.96000 | 5.100 | 1154.520 |

**Table 16**

| QUARTILES OF PNT IN MEN: | | | | |
|---|---|---|---|---|
| PNT [pmol/1] | | | | |
| Percentile Group of PNTpmo1L | N | Median | Minimum | Maximum |
| 1 | 450 | 58.02000 | 3.270 | 70.800 |
| 2 | 451 | 85.88000 | 70.970 | 98.820 |
| 3 | 451 | 118.18000 | 98.880 | 143.940 |
| 4 | 451 | 186.39000 | 144.180 | 1057.360 |
| Total | 1803 | 98.88000 | 3.270 | 1057.360 |

**Table 17**

| **CARDIOVASCULAR DISEASE** | | | | | | | | | |
|---|---|---|---|---|---|---|---|---|---|
| | **HR per 1 SD** | **P-value** | **Quartile 1** | **Quartile 2** | **Quartile 3** | | **Quartile 4** | | **P for trend** |
| All (4362/ 519) | 1.17 (1.07-1.27) | <0.001 | 1.0 (ref) | 1.09 (0.84-1.42) | 1.39 (1.09-1.78) | | 1.37 (1.07-1.75) | | 0.003 |
| Women (25591 / 224) | 1.33 (1.17-1.51) | <0.001 | 1.0 (ref) | 0.91 (0.59-1.41) | 1.58 (108-2.30) | | 1.65 (1.13-241) | | 0.001 |
| Men (1803 /295) | 1.06 (0.95-1.19) | 0.310 | 1.0 (ref) | 1.25 (0.90-1.74) | 1.26 (0.90-1.76) | | 1.21 (0.87-1.69) | | 0.278 |
| | | | | | | | | | |
| **CARDIOVASCULAR MORTALITY** | | | | | | | | | |
| | HR **per 1 SD** | **P-value** | **Quartile 1** | **Quartile 2** | **Quartile 3** | **Quartile 4** | | **P for trend** | |
| All (4362 / 174) | 1.29 (1.12-1.49) | 0.001 | 1.0 (ref) | 0.95 (0.59-1.53) | 1.41 (0.91-2.17) | 1.73 (1.14-2.61) | | 0.003 | |
| Women (2559 / 75) | 1.50 (1.21-1.87) | <0.001 | 1.0 (ref) | 1.02 (0.47-2.22) | 1.53 (0.76-3.08) | 2.18 (1.13-4.20) | | 0.008 | |
| Men (1803 / 99) | 1.16 (0.96-1.41) | 0.132 | 1.0 (ref) | 1.06 (0.58-1.93) | 1.36 (0.76-2.42) | 1.43 (0.82-2.51) | | 0.147 | |

### P-NT and prediction of cardiovascular disease, cardiovascular mortality and all-cause mortality

Among subjects without cardiovascular disease prior to the baseline exam, 519 suffered a first cardiovascular disease event during 14.4 ± 4.4 years of follow-up. After full adjustment for baseline levels of cardiovascular risk factors (age, gender, antihypertensive treatment, systolic blood pressure, body mass index, diabetes mellitus, HDL, LDL and smoking) each SD increase of P-NT was associated with 17% increased risk of incident cardiovascular disease (Table 3). There was a strong interaction between P-NT and female gender (P<0.001) and gender stratified analyses revealed that each SD increase of baseline P-NT was strongly associated with a 33% increased risk of incident cardiovascular disease in women, whereas there was no significant relationship among men (Table 3). Quartile analyses revealed that the top versus the bottom quartile was associated with a 37% increased risk for incident cardiovascular disease in the total population and 65% increased risk in women (Table 3).

Additional adjustment for fasting insulin concentration, i.e. the strongest cross-sectional correlate of P-NT, did not affect the results (not shown).

We then assessed the relationship between total and cardiovascular mortality in the entire population as well as in men and women separately in models fully adjusted for all cardiovascular risk factors. Each SD increase of P-NT was associated with a significant 8% increuse in the risk of all-cause mortality in the total population and a 13% risk of all-cause mortality among women whereas there was no such increased risk related to P-NT in men (Table 3). The excess risk of death in appeared to be mainly accounted for by cardiovascular deaths with 29% per SD increase in the risk of cardiovascular death in the total population and 50% excess risk in females. Female subjects belonging to the top as compared to the bottom quartile of P-NT had a more than 2-fold increased risk of suffering cardiovascular death (Table 3).

Multivariate Cox proportional Hazards models for baseline P-NT versus incidence of cardiovascular disease, all-cause- and cardiovascular mortality

### Head-to-head comparison between P-NT, N-BNP and CRP

In order to compare the statistical strength and the effect estimates on the studied endpoints between P-NT and established plasma biomarkers, we entered P-NT simultaneously with N-BNP and CRP in fully adjusted models (CRP and N-BNP was measured as described by Melander et al., JAMA. 2009;302(1):49-57). As seen below, P-NT performed equally well as N-BNP and CRP for most endpoints in the total population and in females P-NT performed clearly better than N-BNP and CRP (CRP alone was not significant in females). Combining (see Melander et al., JAMA. 2009;302(1):49-57) N-BNP and P-NT further improved the predictive power for CVD in females from 33% HR per 1SD (P-NT alone to 34,8% per 1SD (p <0,001) (combination of P-NT and N-BNP

**Table 18**

| Incident CVD- ALL SUBJECTS | | | | | | | | |
|---|---|---|---|---|---|---|---|---|
| **Variables in the Equation** | | | | | | | | |
| | | | | | | | 95.0% CI for Exp(B) | |
| | B | SE | Wald | df | Sig. | Exp(B) | Lower | Upper |
| AGE | .079 | .010 | 68.888 | 1 | .000 | 1.082 | 1.062 | 1.103 |
| SEX | -.476 | .106 | 20.042 | 1 | .000 | .621 | .505 | .765 |
| AHT_B | .231 | .116 | 3.991 | 1 | .046 | 1.260 | 1.004 | 1.581 |
| SBP B | .015 | .003 | 35.264 | 1 | .000 | 1.015 | 1.010 | 1.020 |
| BMI_B | -.012 | .014 | .775 | 1 | .379 | .988 | .962 | 1.015 |
| DM_B | .544 | .130 | 17.618 | 1 | .000 | 1.723 | 1.336 | 2.221 |
| HDL_B | -.851 | .169 | 25.479 | 1 | .000 | .427 | .307 | .594 |
| LDL_B | .152 | .048 | 9.800 | 1 | .002 | 1.164 | 1.058 | 1.280 |
| current_smoker 0 | .495 | .106 | 21.876 | 1 | .000 | 1.640 | 1.333 | 2.017 |
| **ZLN_PNT** | **.133** | **.046** | **8.336** | **1** | **.004** | **1.142** | **1.044** | **1.250** |
| **ZLN_BNP** | **.132** | **.049** | **7.168** | **1** | **.007** | **1.141.** | **1.036** | **1.257** |
| **ZLN CRP** | **.147** | **.050** | **8.523** | **1** | **.004** | **1.158** | **1.049** | **1.278** |

**Table 19**

| **INCIDENT CVD- FEMALE SUBJECTS** | | | | | | | | |
|---|---|---|---|---|---|---|---|---|
| **Variables in the Equation** | | | | | | | | |
| | | | | | | | 95.0% CI for Exp(B) | |
| | B | SE | Wald | df | Sig. | Exp(B) | Lower | Upper |
| AGE | .080 | .015 | 28.703 | 1 | .000 | 1.084 | 1.052 | 1.116 |
| SEX | | | | 0^{a} | | | | |
| AHT_B | .466 | .168 | 7.664 | 1 | .006 | 1.593 | 1.146 | 2.215 |
| SBP_B | .014 | .004 | 13.454 | 1 | .000 | 1.014 | 1.007 | 1.022 |
| BMI B | -.038 | .019 | 3.908 | 1 | .048 | .962 | .927 | 1.000 |
| DM_B | .925 | .202 | 21.035 | 1 | .000 | 2.522 | 1.699 | 3.745 |
| HDL B | -.926 | .231 | 16.088 | 1 | .000 | .396 | .252 | .623 |
| LDL_B | .116 | .069 | 2.801 | 1 | .094 | 1.123 | .980 | 1.286 |
| current smoker | .740 | .155 | 22.725 | 1 | .000 | 2.095 | 1.546 | 2.840 |
| 0 | | | | | | | | |
| **Z_LNBNP** | **.154** | **.071** | **4.755** | **1** | **.029** | **1.167** | **1.016** | **1.340** |
| **ZLN_CRP** | **.112** | **.077** | **2.123** | **1** | **.145** | **1.119** | **.962** | **1.301** |
| **ZLN PNT** | **.224** | **.070** | **10.217** | **1** | **.001** | **1.251** | **1.091** | **1.435** |

| **Variables in the Equation** | | | | | | | | |
|---|---|---|---|---|---|---|---|---|
| | | | | | | | 95.0% CI for Exp(B) | |
| | B | SE | Wald | df | Sig. | Exp(B) | Lower | Upper |
| AGE | .080 | .015 | 28.703 | 1 | .000 | 1.084 | 1.052 | 1.116 |
| SEX | | | . | 0^{a} | | | | |
| AHT_B | .466 | .168 | 7.664 | 1 | .006 | 1.593 | 1.146 | 2.215 |
| SBP_B | .014 | .004 | 13.454 | 1 | .000 | 1.014 | 1.007 | 1.022 |
| BMI B | -.038 | .019 | 3.908 | 1 | .048 | .962 | .927 | 1.000 |
| DM_B | .925 | .202 | 21.035 | 1 | .000 | 2.522 | 1.699 | 3.745 |
| HDL B | -.926 | .231 | 16.088 | 1 | .000 | .396 | .252 | .623 |
| LDL_B | .116 | .069 | 2.801 | 1 | .094 | 1.123 | .980 | 1.286 |
| current smoker | .740 | .155 | 22.725 | 1 | .000 | 2.095 | 1.546 | 2.840 |
| 0 | | | | | | | | |
| **Z_LNBNP** | **.154** | **.071** | **4.755** | **1** | **.029** | **1.167** | **1.016** | **1.340** |
| Z**LN_CRP** | **.112** | **.077** | **2.123** | **1** | **.145** | **1.119** | **.962** | **1.301** |
| **ZLN PNT** | **.224** | **.070** | **10.217** | **1** | **.001** | **1.251** | **1.091** | **1.435** |

Fig 2: Kaplan Meier Graph for illustrating the cumulative CVD-events in women, cut off >< median = 109 pmol/1P-NT.

The prediction of CVD-events by baseline P-NT was given for the complete observation period.

### Subgroup analysis

Using the same variables in the equation, we investigated different subgroups for prediction of CVD, mortality, CVD mortality. Subjects with precious CVD-events were excluded.

**Table 20**

| Prediction of CVD events | | | | |
|---|---|---|---|---|
| Subgroup | No of subjects | No of events | Hazard risk per 1 SD PNT | Significance (p-value) |
| all | 4361 | 519 | 16,5% | <0,001 |
| women | 2559 | 224 | 33,2% | <0,001 |
| male | 1802 | 295 | 6% | 0,31 (n.s.) |
| Diabetic women | 163 | 40 | 42,9% | 0,05 |
| nonDiabetic women | 2396 | 184 | 33,6% | <0,001 |
| HBP women | 1545 | 178 | 30,6% | <0,001 |
| Normal BP Women | 1014 | 46 | 40,8% | 0,014 |
| Women w/o history of cancer, diabetes and CVD events | 2022 | 144 | 30,6% | 0,001 |

Prediction of CVD events was only related to females. The predictive power of P-NT was similar in completely healthy and in high risk subgroups like diabetic women or HBP women.

**Table 21**

| Prediction of CVD mortality | | | | |
|---|---|---|---|---|
| Subgroup | No of subjects | No of events | Hazard risk per 1SD PNT | Significance (p-value) |
| all | 4361 | 174 | 28,7% | 0,001 |
| women | 2559 | 75 | 50% | <0,001 |
| male | 1803 | 99 | 16% | 0.132 (n.s.) |
| Diabetic women | 163 | 14 | 141% | 0,006 |
| Non Diabetic women) | 2396 | 61 | 39,6% | 0,006 |
| HBP women | 1545 | 63 | 35,1% | 0,016 |
| Normal BP Women | 1014 | 12 | 125,7% | 0,001 |
| Women w/o history of cancer, diabetes and CVD events | 2022 | 48 | 36% | 0,025 |

Prediction of CVD mortality by P-NT was strong in women and not significant in male. The predictive power of P-NT was given in healthy women and in high risk women (diabetic or HBP).

Reclassification of woman into risk groups

### Methods:

We calculated model c-statistics and reclassification across 10-year predicted risk categories for the different events (<5%, >=5-10%, >=10-20% and >=20%, respectively) with Net Reclassification Improvement (NRI) for models with and without P-NT.30-32 All analyses were performed with Stata software version 11 (StataCorp, College Station, Texas). A two-sided P-value of <.05 was considered statistically significant.

For cardiovascular mortality, there was a borderline significant increase of the over-all NRI of 11%. P-NT correctly reclassified 19% of females who actually suffered cardiovascular death to a higher category of risk but only reclassified 5% of women who did not suffer cardiovascular death to a lower category of risk (Table 5). Among women at intermediate (10-20%) 10-year risk, i.e. the group in which biomarker support has been suggested to be particularly important for clinical decision making regarding initiation of primary preventive therapy (and reclassification thus referred to as "clinical NRI"),38 addition of P-NT to traditional cardiovascular risk factors resulted in a significant clinical NRI of 40% for cardiovascular mortality, with reclassification of 21% women who died a cardiovascular death to a higher category of risk and 30% of women who did not suffer cardiovascular death to a lower category of risk.

### Literature:

Pencina MJ, D'Agostino RB. Overall C as a measure of discrimination in survival analysis: model specific population value and confidence interval estimation. Stat Med. Jul 15 2004;23(13):2109-2123.
Pencina MJ, D'Agostino RB, Sr., D'Agostino RB, Jr., Vasan RS. Evaluating the added predictive ability of a new marker: from area under the ROC curve to reclassification and beyond. Stat Med. Jan 30 2008;27(2):157-172; discussion 207-112.
Ridker PM, Buring JE, Rifai N, Cook NR. Development and validation of improved algorithms for the assessment of global cardiovascular risk in women: the Reynolds Risk Score. Jama. Feb 14 2007;297(6):611-619.

### Figure description:

Figure 1 shows a typical P-NT dose/ signal curve
Figure 2: Kaplan Meier Graph for illustrating the cumulative CVD-events in women, cut off >< median = 109 pmol/1 P-N.

## Claims

1. A method for predicting the risk of getting a cardiovascular event in a female subject comprising:
• determining the level of pro-neurotensin 1-117 in a bodily fluid obtained from said female subject; and
• correlating said level of pro-neurotensin 1-117 with the risk of getting a cardiovascular event, wherein an elevated level is predictive of an enhanced risk of getting a cardiovascular event,
wherein said cardiovascular event is an acute cardiovascular event selected from the group comprising myocardial infarction, stroke, acute heart failure and cardiovascular death related to myocardial infarction, stroke or acute heart failure,
and wherein the level of pro-neurotensin 1-117 in a bodily fluid is the fasting level.

2. A method according to claim 1, wherein said female subject has no history of diagnosis of an acute cardiovascular event at the time the sample of bodily fluid is taken from said female subject.

3. A method according to claims 1 to 2, wherein at the time the sample of bodily fluid is taken from said female subject, said female subject has been diagnosed as having a cardiovascular disease or diabetes.

4. A method according to claim 3, wherein the female subject has been diagnosed as having a cardiovascular disease selected from the group comprising heart failure, atherosclerosis, and hypertension.

5. A method according to claim 3, wherein the female subject has been diagnosed as having a diabetes type II.

6. A method according to claims 1 to 5 wherein in addition the level of one or both of the following markers is determined: pro-BNP or fragments or precursors thereof having at least 5 amino acids and/or C-reactive protein (CRP).

7. A method according to claims 1 to 6, wherein additionally at least one clinical parameter is determined selected from the group comprising: age, systolic blood pressure, diastolic blood pressure, antihypertensive treatment, body mass index, presence of diabetes mellitus, and current smoking.

8. A method according to any of claims 1 - 7, wherein said method is performed more than once in order to monitor the risk of getting a cardiovascular event in a female subject.

9. A method according to claim 8, wherein said monitoring is performed in order to evaluate the response of said female subject to preventive and/or therapeutic measures taken.

10. A method according to any of claims 1 to 9 in order to stratify said female subjects into risk groups.

11. Use of a device, e.g. point-of-care device for performing a method according to any of claims 1-10.

## Patentansprüche

1. Verfahren zur Vorhersage des Risikos für ein kardiovaskuläres Ereignis bei einer weiblichen Person, umfassend:
• die Bestimmung des Wertes von Pro-Neurotensin 1-117 in einer von dieser weiblichen Person entnommenen Körperflüssigkeit; und
• die Korrelation dieses Wertes von Pro-Neurotensin 1-117 mit dem Risiko eines kardiovaskulären Ereignisses, wobei ein erhöhter Wert einem erhöhten Risiko für ein kardiovaskuläres Ereignis entspricht,
wobei dieses kardiovaskuläre Ereignis ein akutes kardiovaskuläres Ereignis ist, ausgewählt aus der Gruppe bestehend aus Myokardinfarkt, Schlaganfall, akutes Herzversagen und kardiovaskulärer Tod durch Myokardinfarkt, Schlaganfall oder akutes Herzversagen,
und wobei der Wert von Pro-Neurotensin 1-117 in einer Körperflüssigkeit der nüchterne Wert ist.

2. Verfahren nach Anspruch 1, wobei bei der weiblichen Person zum Zeitpunkt der Entnahme der Körperflüssigkeit keine vorherigen Diagnosen akuter kardiovaskulärer Ereignisse bestehen.

3. Verfahren nach den Ansprüchen 1 bis 2, wobei bei der weiblichen Person zum Zeitpunkt der Entnahme der Körperflüssigkeit eine kardiovaskuläre Erkrankung oder Diabetes diagnostiziert wurde.

4. Verfahren nach Anspruch 3, wobei bei der weiblichen Person eine kardiovaskuläre Erkrankung, ausgewählt aus der Gruppe bestehend aus Herzversagen, Atherosklerose und Hypertonie, diagnostiziert wurde.

5. Verfahren nach Anspruch 3, wobei bei der weiblichen Person Diabetes Typ 2 diagnostiziert wurde.

6. Verfahren nach den Ansprüchen 1 bis 5, wobei zusätzlich der Wert eines oder beider folgender Marker bestimmt wird: pro-BNP oder Fragmente oder Vorstufen davon mit mindestens 5 Aminosäuren und/oder C-reaktives Protein (CRP).

7. Verfahren nach den Ansprüchen 1 bis 6, wobei zusätzlich mindestens ein klinischer Parameter, ausgewählt aus der Gruppe bestehend aus Alter, systolischem Blutdruck, diastolischem Blutdruck, Behandlung mit Antihypertensiva, Body-Mass-Index, Vorhandensein von Diabetes mellitus und derzeitigen Rauchgewohnheiten, bestimmt wird.

8. Verfahren nach einem der Ansprüche 1-7, wobei dieses Verfahren mehr als einmal durchgeführt wird, um das Risiko für ein kardiovaskuläres Ereignis bei einer weiblichen Person zu überwachen.

9. Verfahren nach Anspruch 8, wobei diese Überwachung durchgeführt wird, um die Reaktion der weiblichen Person auf getroffene vorbeugende und/oder therapeutische Maßnahmen zu bewerten.

10. Verfahren nach einem der Ansprüche 1 bis 9, um die besagten weiblichen Personen in Risikogruppen zu stratifizieren.

11. Verwendung einer Vorrichtung, bspw. ein Point-of-Care-Gerät, zur Durchführung eines Verfahrens nach einem der Ansprüche 1-10.

## Revendications

1. Méthode de prédiction du risque de survenue d'un événement cardiovasculaire chez un sujet de sexe féminin, ladite méthode consistant à :
• déterminer le taux de proneurotensine 1-117 dans un fluide corporel obtenu dudit sujet de sexe féminin ; et
• corréler ledit taux de proneurotensine 1-117 avec le risque de survenue d'un événement cardiovasculaire, un taux élevé prédisant un risque accru de survenu d'un événement cardiovasculaire,
ledit événement cardiovasculaire étant un événement cardiovasculaire aigu choisi parmi le groupe comprenant l'infarctus du myocarde, l'accident vasculaire cérébral, l'insuffisance cardiaque aiguë et la mort cardiovasculaire attribuables à l'infarctus du myocarde, à l'accident vasculaire cérébral ou à l'insuffisance cardiaque aigue ;
et le taux de proneurotensine 1-117 dans un fluide corporel étant le taux à jeun.

2. Méthode selon la revendication 1, ledit sujet de sexe féminin n'ayant pas d'antécédents d'événements cardiovasculaires aigües au moment du prélèvement du fluide corporel.

3. Méthode selon les revendications 1 à 2, une maladie cardiovasculaire ou un diabète ayant été diagnostiqué chez ledit sujet de sexe féminin au moment du prélèvement du fluide corporel.

4. Méthode selon la revendication 3, une maladie cardiovasculaire choisie parmi le groupe comprenant l'accident vasculaire, l'athérosclérose et l'hypertension ayant été diagnostiquée chez ledit sujet de sexe féminin.

5. Méthode selon la revendication 3, un diabète de type II ayant été diagnostiqué chez ledit sujet de sexe féminin.

6. Méthode selon l'une des revendications 1 à 5, le taux d'un ou des deux marqueurs suivants étant en outre déterminé : pro-BNP ou ses fragments ou précurseurs ayant au moins 5 acides aminés et/ou la protéine C-réactive (CRP).

7. Méthode selon l'une des revendications 1 à 6, en outre au moins un paramètre clinique déterminé étant choisi parmi le groupe comprenant : âge, tension artérielle systolique, tension artérielle diastolique, traitement antihypertenseur, indice de masse corporelle, présence du diabète sucré et habitudes de tabagisme actuelles.

8. Méthode selon l'une des revendications 1 à 7, ladite méthode étant réalisée plus d'une fois pour surveiller le risque de survenue d'un événement cardiovasculaire chez un sujet de sexe féminin.

9. Méthode selon la revendication 8, ladite surveillance étant réalisée pour évaluer la réponse dudit sujet de sexe féminin à des mesures préventives et/ou thérapeutiques prises.

10. Méthode selon l'une des revendications 1 à 9 pour stratifier lesdits sujets de sexe féminin en groupes de risque.

11. Utilisation d'un dispositif, par exemple un dispositif de diagnostic, pour réaliser une méthode selon l'une des revendications 1 à 10.
